# EUROPEAN PATENT SPECIFICATION

(11) **EP 2 817 285 B1**
(45) Date of publication and mention of the grant of the patent: **09.01.2019**
(21) Application number: 13752071.4
(22) Date of filing: 20.02.2013
(51) Int. Cl.: C07C 51/09, C07C 57/04, C07C 67/37, C07D 303/00, C07D 305/00, C08G 63/08, C08G 63/82, C08F 122/02, G06Q 30/02, C07D 305/12, C08F 120/06

(54) **ACRYLIC ACID PRODUCTION METHODS**
ACRYLSÄUREHERSTELLUNGSVERFAHREN
PROCÉDÉS DE PRODUCTION D'ACIDE ACRYLIQUE

(30) Priority: 22.02.2012 US 201261601707 P; 01.03.2012 US 201261605252 P
(43) Date of publication of application: 31.12.2014
(60) Divisional application: 18208928.4
(73) Proprietor: Novomer, Inc., Boston, Massachusetts 02114 (US)
(72) Inventor: MAHONEY, James, E., Ithaca, NY 14850 (US)
(74) Representative: Tostmann, Holger Carl
(86) International application number: PCT/US2013/026810
(87) International publication number: WO 2013/126375

(56) References cited:
- WO-A1-2010/118128
- WO-A1-2010/118128
- WO-A2-2011/163309
- US-A- 2 361 036
- US-A- 3 002 017
- US-A- 3 002 017
- US-A1- 2005 014 977
- SLOWIK ET AL.: 'Catalytic Conversion of Waste Carbon Monoxide to Valuable Chemicals & Materials' TECHNICAL PROCEEDINGS OF THE 2010 CLEAN TECHNOLOGY CONFERENCE AND TRADE SHOW 2010, pages 283 - 286, XP008175095

## Description

### BACKGROUND OF THE INVENTION

This application claims priority to US Application No. 61/601,707, filed February 22, 2012, and to US Application No. 61/605,252, filed March 1, 2012.

The production and use of acrylic acid (AA) has grown significantly in recent decades as the demand for polyacrylic acid-based superabsorbent polymers (SAPs) has grown. SAPs are used extensively for the manufacture of diapers and in agricultural applications. The successful manufacture of SAPs requires the use of highly pure glacial acrylic acid. Problems arise from the fact that glacial acrylic acid is not stable for storage and transport: the material can undergo unexpected violent polymerization reactions. The polymerization of acrylic acid can be very violent, evolving considerable heat and pressure and ejecting hot vapor and polymer, which may autoignite. An explosion hazard exists due to extremely rapid pressure build up. Several case histories are known in which vessels of acrylic acid exploded due to violent ("runaway") polymerization.

Various techniques have been developed to stabilize glacial AA (see, for example US Patent Nos. 4,480,116; 4,797,504; and 6,403,850) and most commercial AA contains hydroquinone monomethyl ether (MEHQ) and dissolved oxygen for this purpose. Nevertheless, the transport and storage of glacial AA remains problematic. Even with successful stabilization against runaway polymerization, diacrylic acid is formed during storage. The formation of diacrylic acid cannot be prevented by chemical additives and diacrylic acid may adversely affect the performance of acrylic acid in some applications. For these reasons, many processes which use acrylic acid rely upon on-site purification of glacial AA from commercial grade AA. This is an energy intensive process that requires expertise, as well as sophisticated equipment and controls which add to the complexity and cost of processes using glacial AA as a feedstock.

Additionally, recent discoveries of large ethane-rich shale gas reserves in the United States and elsewhere have the potential to impact chemical industries and more specifically, the production of acrylic acid. Currently almost all commercial acrylic acid is derived from propylene oxidation. Propylene is primarily a product of oil refining and its price and availability are closely tied to crude oil prices. Because of this, acrylic acid prices have risen dramatically in recent years.

WO 2011/118128 discloses a method for producing an beta-lactone product. The method includes the steps of: reacting an epoxide, a solvent with a carbonylation catalyst and carbon monoxide to produce a reaction stream comprising a beta-lactone then separating a portion of the beta-lactone in the reaction stream from the solvent and carbonylation catalyst to produce: i) a beta-lactone stream with the beta-lactone, and ii) a catalyst recycling stream including the carbonylation catalyst and the high boiling solvent; and adding the catalyst recycling stream to the feed stream.

US 3 002 017 discloses a method of preparing glacial acrylic acid monomer by depolymerization at a temperature of from about to about 260 C. of a homopolymer of beta-propiolactone, including the step of rapidly quenching vapors of acrylic acid monomer by condensation with sufficient pre-cooled acrylic acid to reduce the temperature below 70 C.

US 2 361 036 discloses a method of preparing alpha-beta unsaturated carboxylic acids particularly the low molecular weight alpha-beta unsaturated monocarboxylic acids such as acrylic acid and substituted acrylic acids. More specifically, US 2 361 036 relates to the polymerization of lactones of beta hydroxy carboxylic acids, and to the pyrolysis of the polymerization products so produced.

WO 2011/163309 discloses, among others, tandem carbonylation and polymerization reactions, where epoxides are catalytically carbonylated to form lactones and the lactones catalytically polymerized to form polymers without the need to isolate or purify the lactone intermediate. Also disclosed are methods for catalytically polymerizing beta-propiolactone.

### SUMMARY OF THE INVENTION

There remains a need for methods to transport and/or store glacial acrylic acid (AA) in a safe and/or energy efficient manner. Additionally, there remains a need for methods to provide an alternative to route to AA that does not rely on propylene oxidation.

In one aspect, the present invention provides a solution to the problems inherent in the storage and transportation of glacial acrylic acid.

In one aspect, the present invention enables a less expensive feedstock to be used for acrylic acid production.

In one aspect, the present invention provides the ability to utilize a less expensive feedstock at one site to satisfy broader geographic demand for acrylic acid and its derivatives. For example, the present invention can be deployed to utilize the C2 component of shale gas and carbon monoxide to make the polymer polypropiolactone (PPL).

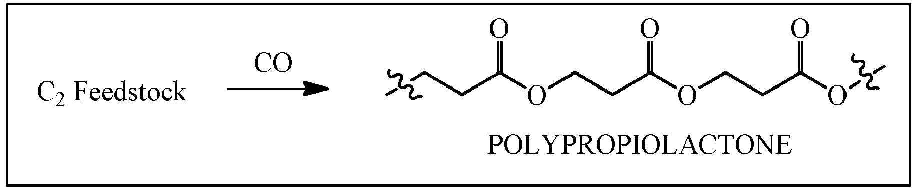

PPL is a stable material that can be safely transported and stored for extended periods without the safety concerns or the quality declines attendant with shipping and storing glacial AA. When glacial acrylic acid is needed, methods of the present invention provide it in highly pure form via a step of decomposing the polypropiolactone at the point of AA use. Therefore, in certain embodiments the present invention enables access to acrylic acid in a safe and/or less expensive and/or highly flexible fashion.

In certain embodiments, a method of the present invention includes the steps of:
- forming polypropiolactone at a first location;
- isolating the polypropiolactone;
- transporting the isolated polypropiolactone to a second location;
- optionally storing the polypropiolactone in inventory until acrylic acid is needed; and
- pyrolyzing the polypropiolactone to liberate acrylic acid.

In accordance with claim 1, the present invention provides a method for producing acrylic acid, the method comprising the steps of: forming polypropiolactone at a first location, comprising contacting beta-propiolactone with a polymerization catalyst comprising an acrylate anion; isolating at least some of the polypropiolactone; and pyrolyzing at least some of the isolated polypropiolactone to liberate acrylic acid at a second location.

In accordance with claim 3, the present invention provides a method for producing acrylic acid, the method comprising the steps of: forming polypropiolactone at a first location, receiving, at a second location, polypropiolactone formed at a first location; and pyrolyzing at least some of the received polypropiolactone to liberate acrylic acid at the second location.

### BRIEF DESCRIPTION OF THE DRAWINGS

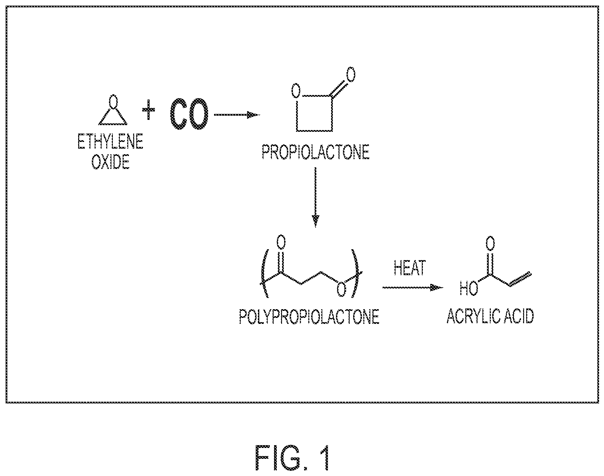

- FIG. 1: shows a schematic of certain embodiments of the present invention.

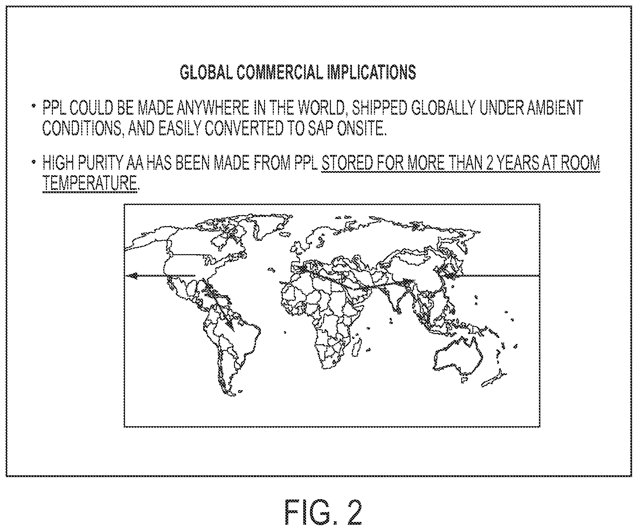

- FIG. 2: shows exemplary first and second locations according to certain embodiments of the present invention.
- FIG. 3: shows an embodiment of the invention wherein the step of transporting the polypropiolactone to a second location comprises the substeps of forming a thermoplastic propiolactone composition into a useful article which can be marketed to a consumer, and collecting the useful article as a post-consumer recycling stream which can then be treated as described herein to provide acrylic acid.
- FIG. 4: shows a ¹H NMR spectrum of a sample of polypropiolactone useful for practicing the present invention.

### DETAILED DESCRIPTION OF THE INVENTION

In accordance with claim 1, the present invention provides a method for producing acrylic acid, the method comprising the steps of: forming polypropiolactone at a first location comprising contacting beta-propiolactone with a polymerization catalyst comprising an acrylate anion; isolating at least some of the polypropiolactone; and pyrolyzing at least some of the isolated polypropiolactone to liberate acrylic acid at a second location.

In accordance with claim 2, the method further includes the step of transporting the isolated polypropiolactone to the second location prior to pyrolyzing at least some of the isolated polypropiolactone to liberate acrylic acid.

In accordance with claim 3, the present invention provides a method for producing acrylic acid, the method comprising the steps of: forming polypropiolactone at a first location comprising contacting beta-propiolactone with a polymerization catalyst comprising an acrylate anion; receiving at a second location polypropiolactone formed at a first location; and pyrolyzing at least some of the received polypropiolactone to liberate acrylic acid at the second location.

In certain embodiments, the method includes the step of storing the polypropiolactone prior to pyrolyzing at least some of the isolated polypropiolactone to liberate acrylic acid. The step of storing the polypropiolactone can occur at the first location, at the second location, at one or more other locations (e.g., during transportation), or at any combination of these locations. In certain embodiments, the polypropiolactone is stored at the first location prior to transporting it from the first location. In certain embodiments, the polypropiolactone is stored at the second location prior to pyrolyzing at least some of it. In certain embodiments, the polypropiolactone is stored for at least 1 week, for at least 1 month, for at least 6 months, for at least 1 year, or for at least 2 years.

Price differences between different locations can make it advantageous to form polypropiolactone at one location, and pyrolyze polypropiolactone to liberate acrylic acid at a different location. The ability to safely store and transport polypropiolactone enables the formation of polypropiolactone at a first location where the cost of raw materials is less than at a second location, followed by transportation to the second location and subsequent pyrolysis to liberate acrylic acid.

In certain embodiments, methods of the present invention are characterized in that the location where the polypropiolactone is produced (i.e. the first location) and the location where at least a portion of the polypropiolactone is pyrolyzed (i.e. the second location) are at least 100 miles apart. In certain embodiments, the first location and the second location are between 100 and 12,000 miles apart. In certain embodiments, the first location and the second location are at least, 250 miles, at least 500 miles, at least 1,000 miles, at least 2,000 or at least 3,000 miles apart. In certain embodiments, the first location and the second location are between about 250 and about 1,000 miles apart, between about 500 and about 2,000 miles apart, between about 2,000 and about 5,000 miles apart, or between about 5,000 and about 10,000 miles apart. In certain embodiments, the first location and the second location are in different countries. In certain embodiments, the first location and the second location are on different continents.

In certain embodiments where methods of the present invention include the step of transporting polypropiolactone from a first location to a second location, the step of transporting comprises moving the polypropiolactone a distance of more than 100 miles. In certain embodiments, the step of transporting comprises moving the polypropiolactone a distance of more than 500 miles, more than 1,000 miles, more than 2,000 miles or more than 5,000 miles. In certain embodiments, the step of transporting comprises moving the polypropiolactone a distance of between 100 and 12,000 miles. In certain embodiments, the step of transporting comprises moving the polypropiolactone a distance of between about 250 and about 1000 miles, between about 500 and about 2,000 miles, between about 2,000 and about 5,000 miles, or between about 5,000 and about 10,000 miles. In certain embodiments, the step of transporting comprises moving the polypropiolactone from a first country to a second country. In certain embodiments, the step of transporting comprises moving the polypropiolactone from a first continent to a second continent.

In certain embodiments, the step of transporting comprises moving the polypropiolactone from the North America to Europe. In certain embodiments, the step of transporting comprises moving polypropiolactone from the North America to Asia. In certain embodiments, the step of transporting comprises moving the polypropiolactone from the US to Europe. In certain embodiments, the step of transporting comprises moving polypropiolactone from the US to Asia. In certain embodiments, the step of transporting comprises moving polypropiolactone from the Middle East to Asia. In certain embodiments, the step of transporting comprises moving polypropiolactone from the Middle East to Europe. In certain embodiments, the step of transporting comprises moving polypropiolactone from Saudi Arabia to Asia. In certain embodiments, the step of transporting comprises moving polypropiolactone from Saudi Arabia to Europe.

In certain embodiments, the step of transporting comprises moving the polypropiolactone by a means selected from: truck, train, tanker, barge, ship, and combinations of any two or more of these. In certain embodiments, the method includes the steps as described above wherein, on a predetermined day, the price of ethylene at the first location is less than the price of ethylene at the second location. In certain embodiments, the method includes the steps as described above wherein, on a predetermined day, the price of ethylene at the first location is less than the price of propylene at the second location. In certain embodiments, the method includes the steps as described above wherein, on a predetermined day, the price of the C2 component of shale gas at the first location is less than the price of ethylene at the second location. In certain embodiments, the method includes the steps as described above wherein, on a predetermined day, the price of the C2 component of shale gas at the first location is less than the price of propylene at the second location. In certain embodiments, the method includes the steps as described above wherein, on a predetermined day, the price of ethane at the first location is less than the price of ethane at the second location. In certain embodiments, the method includes the steps as described above wherein, on a predetermined day, the price of ethane at the first location is less than the price of propane at the second location. The predetermined day can be any day between 15 and 365 days inclusive, between 15 and 180 days inclusive, between 30 and 90 days inclusive, between 30 and 60 days inclusive, or between 60 and 90 days inclusive prior to the day on which forming the polypropiolactone occurs.

The price differences between different locations can arise because of the first location's access to ethane from a shale play or basin. Access can be via physical proximity to the shale gas, or via access to a pipeline providing shale gas. In certain embodiments, the price differences between different locations arise because of the first location's physical proximity to a shale play or basin. In certain embodiments, the first location is characterized in that it is located within 600 miles, 450 miles, 300 miles or 150 miles of a shale play or basin. See, e.g., Platts World Shale Resources Map.

It will be recognized by those skilled in the art that such materials have reported prices (e.g., a daily price), even if the price fluctuates throughout the specified period (e.g., a day), and that is the price that is intended. Such prices can be found by reference to commercial sources, e.g., Platts (including ethylene, propylene), ICIS (including ethylene, propylene). It will similarly be recognized by those skilled in the art that such materials have reported prices for areas that may be defined by geographic and/or political and/or other considerations (e.g., individual nations such as China, the United States, Saudi Arabia or Brazil, or larger regions such as Northwest Europe, or smaller regions), and one skilled in the art will understand for any given location which is the appropriate price to consult.

Because of such price differentials, it can be advantageous to export polypropiolactone from the first location to a party intending to pyrolyze at least some of the polypropiolactone to liberate acrylic acid at the second location. Thus, in certain embodiments, the present invention provides a method including the steps of: forming polypropiolactone at a first location; isolating at least some of the polypropiolactone; and dispatching at least some of the isolated polypropiolactone to a second location for pyrolysis to liberate acrylic acid. The dispatching can take the form of any action intended to deliver the polypropiolactone ultimately for pyrolysis to acrylic acid (e.g., transporting, exporting, offering for sale).

In certain embodiments, the method is characterized in that the liberated acrylic acid is glacial acrylic acid. In certain embodiments, the liberated glacial acrylic acid is of a purity suitable for direct use in the manufacture of acrylic acid polymers such as SAPs.

In certain embodiments, the polypropiolactone produced in the first step is characterized in that it is a liquid. In certain embodiments, such liquid polypropiolactone compositions have a significant amount of relatively low-molecular weight oligomers. In certain embodiments, the number average molecular weight (M_{N}) of the polypropiolactone produced is between about 200 g/mol and about 10,000 g/mol. In certain embodiments, the M_{N} of the polypropiolactone produced is less than about 5,000 g/mol, less than about 3,000 g/mol, less than about 2,500 g/mol, less than about 2,000 g/mol, less than about 1,500 g/mol, less than about 1,000 g/mol, or less than about 750 g/mol. In certain embodiments, the polypropiolactone produced comprises oligomers containing from 2 to about 10 monomer units. In certain embodiments, such oligomers comprise cyclic oligomers. In certain embodiments, cyclic oligomers contain, on average about 2 monomer units, about 3 monomer units, about 4 monomer units, about 5 monomer units, about 6 monomer units, up to about 10 monomer units, or mixtures of two or more of these materials.

In certain embodiments, the polypropiolactone produced in the first step is characterized in that it is a solid. In certain embodiments, the method includes the additional step of pelletizing the solid polypropiolactone such that it can be easily handled in bulk. In certain embodiments, solid polypropiolactone compositions comprise a significant percentage of high molecular weight polymer chains. In certain embodiments, such high molecular polypropiolactone is characterized in that it has an M_{N} between about 10,000 g/mol and about 1,000,000 g/mol. In certain embodiments, high molecular polypropiolactone is characterized in that it has an M_{N} greater than about 10,000 g/mol, greater than about 20,000 g/mol, greater than about 50,000 g/mol, greater than about 70,000 g/mol, greater than about 100,000 g/mol, greater than about 150,000 g/mol, greater than about 200,000 g/mol, or greater than about 300,000 g/mol.

In certain embodiments, the step of forming the polypropiolactone comprises a step of polymerizing beta propiolactone (BPL). The polymerization may be accomplished by contacting BPL with carboxylate polymerization initiators. The initiation process covalently incorporates such carboxylates into the polymer chain. In certain embodiments, the present invention provides a solution to a potentially undesirable effect of this bound initiator: namely, when the PPL is depolymerized to provide acrylic acid, the carboxylic acid corresponding to the polymerization initiator may also be liberated and may act as a contaminant in the acrylic acid produced. Therefore, in certain embodiments, the step of polymerizing the BPL comprises contacting the BPL with a polymerization catalyst comprising an acrylate anion. Such polymers have the advantage that no non-acrylate materials arising from the bound initiator will contaminate the subsequent acrylic acid stream produced from the polymer.

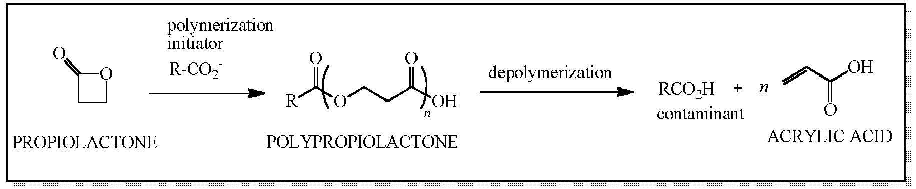

In certain embodiments, the step of polymerizing the BPL comprises contacting BPL with a polymerization catalyst comprising an anion of a non-volatile material. In certain embodiments, PPL made with such non-volatile initiators are desirable because they produce fewer volatile byproducts which may contaminate the acrylic acid stream produced. In certain embodiments, a non-volatile initiator used in such embodiments comprises a polyacid. In certain embodiments, a polyacid comprises a polymeric material, or an acid-functionalized solid. In certain embodiments, a polyacid comprises a polycarboxylic acid. In certain embodiments, a polyacid comprises a sulfonic acid. In certain embodiments, a polyacid comprises both carboxylic and sulfonic acid groups.

In certain embodiments, the step of forming the polypropiolactone comprises a step of reacting ethylene oxide with carbon monoxide. In certain embodiments, the step of forming the polypropiolactone comprises the step of carbonylating ethylene oxide to provide propiolactone which is then polymerized to provide PPL. In certain embodiments, the BPL is not isolated and is polymerized *in situ* to provide the PPL.

In certain embodiments, the step of forming the polypropiolactone comprises performing an alternating copolymerization of ethylene oxide and carbon dioxide.

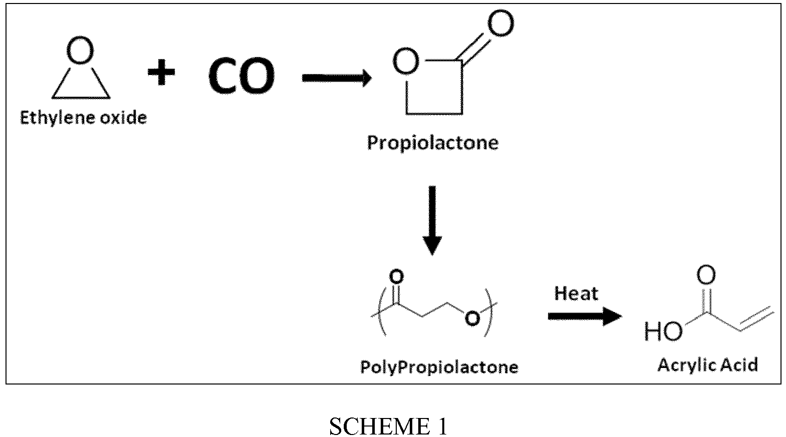

In certain embodiments, the step of pyrolyzing the polypropiolactone, comprises heating the PPL to a temperature of greater than 100 °C, greater than 150 °C, greater than 175 °C, greater than 200 °C, or greater than about 220 °C. In certain embodiments, the step of pyrolyzing the polypropiolactone comprises heating the PPL in an inert atmosphere. In certain embodiments, the step of pyrolyzing the polypropiolactone comprises heating the PPL under a reduced pressure. In certain embodiments, the step of pyrolyzing the polypropiolactone comprises heating the PPL in the presence of a depolymerization catalyst.

In certain embodiments, methods of the present invention include the additional step of isolating the acrylic acid from the pyrolysis step. In certain embodiments, the step of isolating the acrylic acid comprises condensing the acid from a gaseous stream released from the pyrolysis step. In certain embodiments, the acrylic acid is not isolated, but is introduced directly into a polymerization reactor where it is polymerized to polyacrylic acid (e.g. by anionic or radical olefin polymerization methods.)

In certain embodiments, the step of pyrolyzing the PPL is performed continuously (e.g. in a fed batch reactor or other continuous flow reactor format). In certain embodiments, the continuous pyrolysis process is linked to a continuous polymerization process to provide AA at a rate matched to the consumption rate of the reactor. In certain embodiments, this method has the advantage of not requiring the addition and/or removal of stabilizers to or from the acrylic acid feed of the polymerization reactor.

In certain embodiments, the step of transporting the polypropiolactone to a second location comprises the substeps of:
- forming a thermoplastic propiolactone composition into a useful article which can be marketed to a consumer, and
- collecting the useful article as a post-consumer recycling stream.

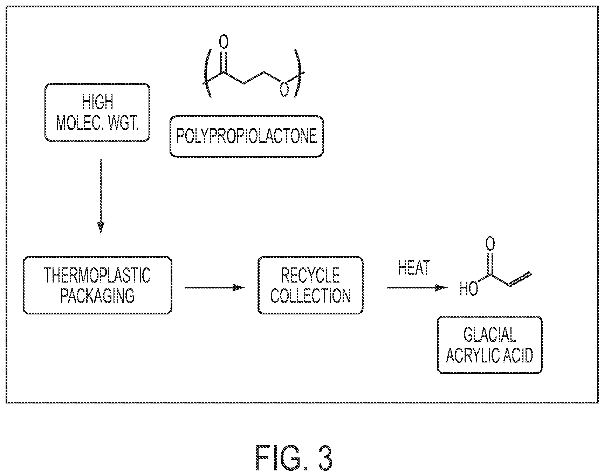

The recycle stream can then be treated as described above to provide acrylic acid. FIG. 3 shows a schematic of such an embodiment.

Therefore, in certain embodiments, the present invention encompasses a method comprising the steps of:
- forming a polypropiolactone polymer;
- manufacturing a useful article comprising the polypropiolactone;
- collecting the article comprising the polypropiolactone as a post-consumer recycling stream; and
- pyrolyzing the polypropiolactone to liberate acrylic acid.

In certain embodiments, the step of manufacturing a useful article from the polypropiolactone comprises making a consumer packaging item. In certain embodiments, a consumer packaging item comprises a bottle, a disposable food container, a foamed article, a blister pack or the like. In certain embodiments, the useful article comprises a film, such an agricultural film, or a packaging film. In certain embodiments, the useful article comprises a molded plastic article such as eating utensils, plastic toys, coolers, buckets, a plastic component in a consumer product such as electronics, automotive parts, sporting goods and the like. In certain embodiments a useful article comprises any of the myriad of articles presently made from thermoplastics such as polyethylene, polypropylene, polystyrene, PVC and the like. In certain embodiments, the useful article comprises a fiber or a fabric.

In certain embodiments, the steps of collecting the article comprising the polypropiolactone as a post-consumer recycling stream; and pyrolyzing the polypropiolactone to liberate acrylic acid, include one or more additional sub-steps such as separating polypropiolactone components from non-polypropiolactone components; shredding, grinding, or melting the articles comprising the polypropiolactone; drying the shredded, ground or melted material; and/or treating polypropiolactone-containing material to remove non- polypropiolactone components such as colorants, fillers, additives and the like prior to the pyrolysis step.

In certain embodiments, the step of collecting the article comprising the polypropiolactone as a post-consumer recycling stream includes the step of providing an article with indicia to convey to a consumer or a recycling facility that the material comprises polypropiolactone. In certain embodiments, such indicia comprise a number indicator which is associated with PPL. In certain embodiments, the indicia comprise an SPI (Society of the Plastics Industry) recycling code.

### EXEMPLIFICATION

The following examples provide non-limiting technical details of certain aspects of the present invention.

### Examples 1-3: Laboratory-scale preparations of acrylic acid from Ethylene Oxide via Polypropiolactone

In this example, one chemical sequence having utility in methods of the present invention is performed at small laboratory scale.

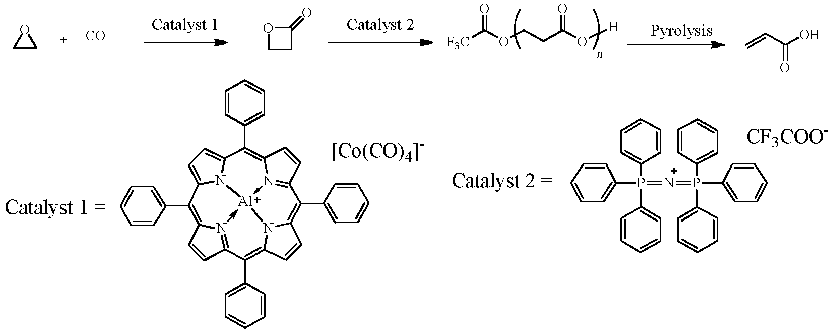

### Step 1: Carbonylation of EO and Polymerization of BPL.

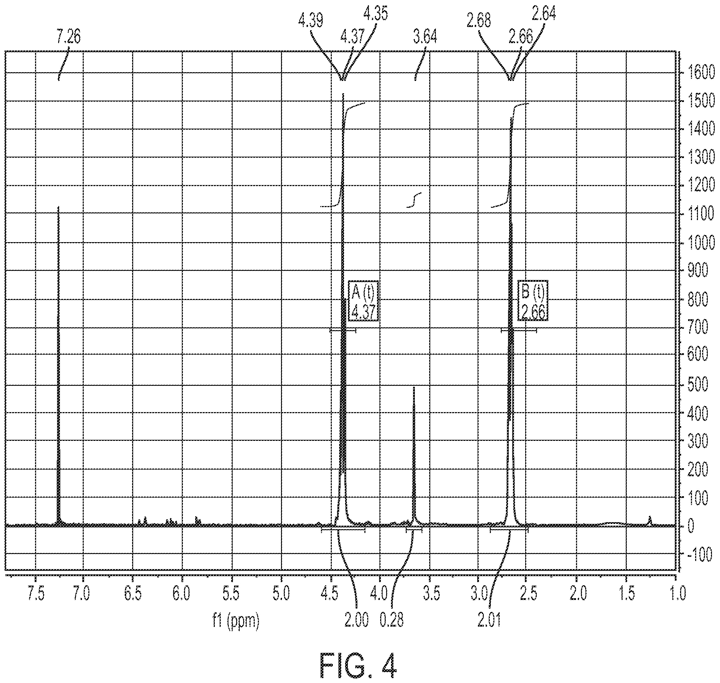

Under dry nitrogen, a 300 mL Parr high-pressure reactor was charged with catalyst 1 ([(TPP)Al(THF)₂][Co(CO)₄], 286 mg, 0.3 mmol) and 85 mL of dry, deoxygenated THF. The reactor was heated to 45 °C, agitated at 500 rpm, and pressurized to 150 psi with CO. After the reactor temperature stabilized, 13.5 g of EO (306 mmol) was injected under 600 psi of CO. the reaction mixture was maintained at 600 psi for 210 min after EO injection, then the CO pressure was slowly vented to ambient pressure. A solution of catalyst 2 was then added to the reactor (PPNTFA, 1.98 g 3.0 mmol in 5 mL of methylene chloride) under nitrogen. The mixture was stirred in the reactor at 45 °C for 16 hours. The polymerization was quenched by addition of 33 mL of 1% HCl in MeOH. 250 mL of MeOH was then added to precipitate the polymer. The reactor was emptied and washed with 20 mL of CHCl₃. The collected reaction mixture and the wash were combined, and filtered to yield a white solid. The solid was washed with 100 mL of MeOH, dissolved in 40 mL of CHCl₃ and re-precipitated in 300 mL of MeOH. The precipitate was filtered washed with 200 mL of MeOH and dried in vacuum oven at 40 °C for 16 hours, to provide 15.51 g of PPL. A proton NMR spectrum (CDCl₃) of the polymer is shown in FIG. 4.

### Step 2: pyrolysis of polypropiolactone

In a 50 mL round bottom flask, 10 g of sand, 2.0 g of poly(propiolactone) from Step 1, and 8.6 mg of MEHQ (hydroquinone monomethyl ether) were combined, and the mixture stirred with a magnetic stir bar. The flask was connected to another 50 mL round bottom flask containing 8.4 mg of MEHQ by a transfer adapter bridge. The whole system was set under vacuum, and was closed when the pressure reached 500 mTorr. The flask containing the polymer was then placed in a heating mantle, and heated to 210 °C, while the receiving flask was immersed in dry ice/acetone bath. Acrylic acid was liberated from pyrolysis of the polymer in the heated flask and was vacuum transferred to the receiving flask. Heating was stopped when no additional liquid was condensing in the receiving flask. At the end of the pyrolysis, 1.39 g of clear liquid was recovered from the receiving flask. GC analysis of the liquid showed that the liquid to be acrylic acid of at least 99.4% purity.

### Example 2: Use of Acrylate as the Polymerization Initiator

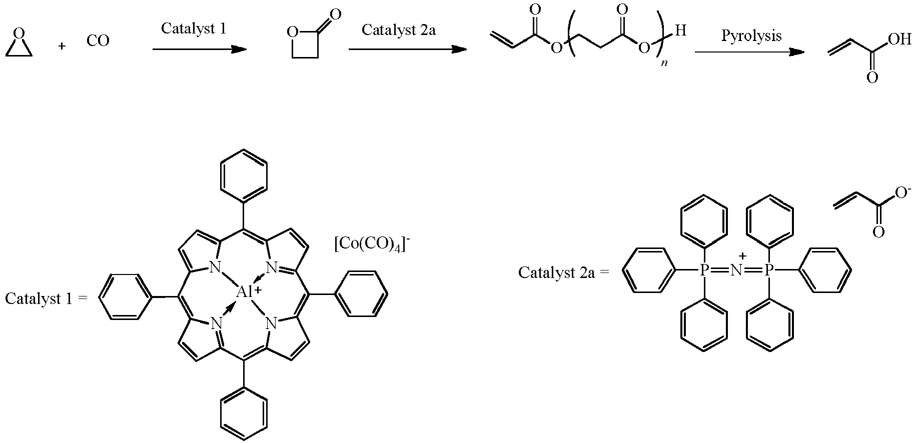

This example is performed under the conditions described in Example 1, except PPN acrylate is used as the polymerization catalyst. The polypropiolactone produced contains acrylate end groups and its pyrolysis liberates only acrylic acid.

### Example 3: Storage of Polypropiolactone as Stable Acrylic Acid Precursor

This example is performed under the conditions described in Example 1, except the polypropiolactone is stored in air at room temperature for 1 year before pyrolysis. The yield and quality of the acrylic acid produced are unchanged from Example 1.

### Example 4: Pilot scale implementation of Acrylic Acid Supply Chain.

In this example, a supply chain innovation of the present invention is demonstrated at pilot scale.

A first reactor proximate to a shale gas play is fed with 75 kg/hr of ethylene oxide derived from a shale gas-derived C2 product stream. The first reactor is operated at steady state conditions with a 1.5 M concentration of beta propiolactone present in the reactor volume. Additionally, 4850 L/hr of solvent containing 15 mol/hr of catalyst 1 [(TPP)Al(THF)₂][Co(CO)₄] is fed to the reactor. The reactor is maintained at a pressure of 600 psig of carbon monoxide and sized such that the feed and solvent have a residence time of at least 2.5 hours, (e.g., at least 15,000 L in volume). Under these conditions, a reaction stream containing about 1740 mole/hr of beta-propiolactone is produced (125 kg/hr).

The beta-lactone stream is directed to a separation unit which separates the stream into a catalyst recycling stream containing solvent and catalyst and a beta propiolactone stream comprising propiolactone and solvent. The catalyst recycling stream is returned to the first reactor and the beta propiolactone stream is fed to a second reactor where it is contacted with PPN-acrylate (catalyst 2a). The second reactor is a plug flow reactor sized such that reactants have a residence time of at least 30 minutes (e.g., 1250 L in volume) maintained at a temperature and catalyst load such that all of the lactone is consumed during the residence time. The second reactor produces approximately 1740 mole/hr of polypropiolactone (123 kg/hr). The effluent of the plug flow reactor is treated with hydrochloric acid and methanol to precipitate the polymer. The precipitated polymer is pelletized and offered for sale as an acrylic acid precursor.

The pellets are transferred 1,500 miles by cargo ship to the facility of an acrylic acid end-user where they are stored in inventory.

The inventory is used to feed a hopper joined to a fluidized bed reactor. The fluidized bed reactor is swept with dry nitrogen at 150 °C and fed from the hopper at a rate of 500 kg of polypropiolactone pellets per hour. The nitrogen sweep from the fluidized bed is directed to a condenser stage which produces a stream of liquid glacial acrylic acid at a rate of approximately 480 kg/hr.

## Claims

1. A method for producing acrylic acid, the method comprising the steps of:
forming polypropiolactone at a first location, comprising contacting beta-propiolactone with a polymerization catalyst comprising an acrylate anion;
isolating at least some of the polypropiolactone; and
pyrolyzing at least some of the isolated polypropiolactone to liberate acrylic acid at a second location.

2. The method of claim 1, further comprising the step of transporting the isolated polypropiolactone to the second location prior to said pyrolyzing, preferably wherein the step of transporting comprises moving the polypropiolactone a distance of more than 100 miles.

3. A method for producing acrylic acid, the method comprising the steps of:
forming polypropiolactone at a first location, comprising contacting beta-propiolactone with a polymerization catalyst comprising an acrylate anion;
receiving, at a second location, polypropiolactone formed at said first location, and pyrolyzing at least some of the received polypropiolactone to liberate acrylic acid at the second location.

4. The method of any of claims 1 to 3, further comprising the step of storing the polypropiolactone prior to said pyrolyzing.

5. The method of any of claims 1 to 3, wherein the first location and the second location are between 100 and 12,000 miles apart, preferably: wherein the first location and the second location are at least 250 miles, at least 500 miles, at least 1,000 miles, at least 2,000 or at least 3,000 miles apart.

6. The method of claim 4, wherein (A) the step of storing the polypropiolactone occurs at the first location, or:
wherein (B) the step of storing the polypropiolactone occurs at the second location.

7. The method of claim 4, wherein the step of storing comprises storing the polypropiolactone for at least 1 week.

8. The method of claim 7, wherein (A) the step of storing comprises storing the polypropiolactone for at least 1 month, or:
wherein (B) the step of storing comprises storing the polypropiolactone for at least 6 months.

9. The method of any one of claims 1 to 8, wherein the betapropiolactone is formed by reacting ethylene oxide with carbon monoxide.

10. The method of any of claims 1 to 9, **characterized in that** the acrylic acid liberated by the pyrolysis is glacial acrylic acid.

11. The method of any of claims 1 to 10, comprising the additional step of feeding the acrylic acid liberated by the pyrolysis to an acrylic acid polymerization, preferably wherein the acrylic acid is fed directly to the acrylic acid polymerization without isolation and storage of the acrylic acid, further preferably wherein a rate of the pyrolysis is matched to a rate of polymerization in the acrylic acid polymerization.

## Patentansprüche

1. Verfahren zur Herstellung von Acrylsäure, wobei das Verfahren folgende Schritte umfasst:
Bilden von Polypropiolacton an einem ersten Ort, umfassend ein In-Kontakt-Bringen von Beta-Propiolacton mit einem Polymerisationskatalysator, der ein Acrylat-Anion umfasst;
Isolieren von mindestens etwas des Polypropiolactons; und
Pyrolysieren von mindestens etwas des isolierten Polypropiolactons, um Acrylsäure an einem zweiten Ort freizusetzen.

2. Verfahren nach Anspruch 1, weiterhin umfassend den Schritt eines Transportierens des isolierten Polypropiolactons an den zweiten Ort vor dem Pyrolysieren, vorzugsweise wobei der Schritt des Transportierens ein Bewegen des Polypropiolactons über eine Entfernung von mehr als 100 Meilen umfasst.

3. Verfahren zum Herstellen von Acrylsäure, wobei das Verfahren folgende Schritte umfasst:
Bilden von Polypropiolacton an einem ersten Ort, umfassend ein In-Kontakt-Bringen von Beta-Propiolacton mit einem Polymersationskatalysator, der ein Acrylat-Anion umfasst;
Erhalten, an einem zweiten Ort, von Polypropiolacton, gebildet an dem ersten Ort, und Pyrolysieren von mindestens etwas des erhaltenen Polypropiolactons, um Acrylsäure an dem zweiten Ort freizusetzen.

4. Verfahren nach einem der Ansprüche 1 bis 3, weiterhin umfassend den Schritt eines Lagerns des Polypropiolactons vor dem Pyrolysieren.

5. Verfahren nach einem der Ansprüche 1 bis 3, wobei der erste Ort und der zweite Ort zwischen 100 und 12.000 Meilen voneinander entfernt liegen, vorzugsweise: wobei der erste Ort und der zweite Ort mindestens 250 Meilen, mindestens 500 Meilen, mindestens 1.000 Meilen, mindestens 2.000 oder mindestens 3.000 Meilen voneinander entfernt liegen.

6. Verfahren nach Anspruch 4, wobei (A) der Schritt des Lagerns des Polypropiolactons an dem ersten Ort stattfindet, oder:
wobei (B) der Schritt des Lagerns des Polypropiolactons an dem zweiten Ort stattfindet.

7. Verfahren nach Anspruch 4, wobei der Schritt des Lagerns ein Lagern des Polypropiolactons für mindestens 1 Woche umfasst.

8. Verfahren nach Anspruch 7, wobei (A) der Schritt des Lagerns ein Lagern des Polypropiolactons für mindestens 1 Monat umfasst, oder:
wobei (B) der Schritt des Lagerns ein Lagern des Polypropiolactons für mindestens 6 Monate umfasst.

9. Verfahren nach einem der Ansprüche 1 bis 8, wobei das Beta-Propiolacton durch Umsetzen von Ethylenoxid mit Carbonmonoxid gebildet wird.

10. Verfahren nach einem der Ansprüche 1 bis 9, **dadurch gekennzeichnet, dass** die Acrylsäure, die durch die Pyrolyse freigesetzt wird, Eisacrylsäure ist.

11. Verfahren nach einem der Ansprüche 1 bis 10, umfassend den zusätzlichen Schritt eines Zuführens der Acrylsäure, die durch die Pyrolyse freigesetzt wird, einer Acrylsäurepolymerisation, vorzugsweise wobei die Acrylsäure der Acrylsäurepolymerisation direkt zugeführt wird ohne Isolierung und Lagerung der Acrylsäure, weiterhin vorzugweise wobei eine Geschwindigkeit der Pyrolyse an eine Polymerisationsgeschwindigkeit bei der Acrylsäurepolymerisation angepasst wird.

## Revendications

1. Procédé pour produire de l'acide acrylique, le procédé comprenant les étapes suivantes :
formation de polypropiolactone à un premier emplacement, comprenant la mise en contact de bêta-propiolactone avec un catalyseur de polymérisation comprenant un anion acrylate ;
isolation d'au moins une partie de la polypropiolactone ; et
pyrolyse d'au moins une partie de la polypropiolactone isolée pour libérer de l'acide acrylique à un deuxième emplacement.

2. Procédé selon la revendication 1, comprenant en outre l'étape de transport de la polypropiolactone isolée vers le deuxième emplacement avant ladite hydrolyse, de préférence dans lequel l'étape de transport comprend le déplacement de la polypropiolactone à une distance supérieure à 100 miles.

3. Procédé pour produire de l'acide acrylique, le procédé comprenant les étapes suivantes :
formation de polypropiolactone en un premier emplacement, comprenant la mise en contact de bêta-propiolactone avec un catalyseur de polymérisation comprenant un anion acrylate ;
réception, en un deuxième emplacement, de la polypropiolactone formée audit deuxième emplacement ; et
pyrolyse d'au moins une partie de la polypropiolactone isolée pour libérer de l'acide acrylique au deuxième emplacement.

4. Procédé selon l'une quelconque des revendications 1 à 3, comprenant en outre l'étape de stockage de la polypropiolactone avant ladite pyrolyse.

5. Procédé selon l'une quelconque des revendications 1 à 3, dans lequel le premier emplacement et le deuxième emplacement sont espacés de 100 à 12 000 miles, de préférence dans lequel le premier emplacement et le deuxième emplacement sont espacés d'au moins 250 miles, d'au moins 500 miles, d'au moins 1 000 miles, d'au moins 2 000 miles ou d'au moins 3 000 miles.

6. Procédé selon la revendication 4, dans lequel (A) l'étape de stockage de la polypropiolactone a lieu au premier emplacement ; ou
dans lequel (B) l'étape de stockage de la polypropiolactone a lieu au deuxième emplacement.

7. Procédé selon la revendication 4, dans lequel l'étape de stockage comprend le stockage de la polypropiolactone pendant au moins 1 semaine.

8. Procédé selon la revendication 7, dans lequel (A) l'étape de stockage comprend le stockage de la polypropiolactone pendant au moins 1 mois ; ou
dans lequel (B) l'étape de stockage comprend le stockage de la polypropiolactone pendant au moins 6 mois.

9. Procédé selon l'une quelconque des revendications 1 à 8, dans lequel la bêta-propiolactone est formée par réaction d'oxyde d'éthylène avec du monoxyde de carbone.

10. Procédé selon l'une quelconque des revendications 1 à 9, **caractérisé en ce que** l'acide acrylique libéré par la pyrolyse est de l'acide acrylique glacial.

11. Procédé selon l'une quelconque des revendications 1 à 10, comprenant l'étape additionnelle d'introduction de l'acide acrylique libéré par la pyrolyse dans une polymérisation d'acide acrylique, de préférence dans lequel l'acide acrylique est introduit directement dans la polymérisation d'acide acrylique sans isolation et stockage de l'acide acrylique, en outre de préférence dans lequel la vitesse de pyrolyse est accordée à la vitesse de polymérisation dans la polymérisation d'acide acrylique.
